# EUROPÄISCHE PATENTSCHRIFT

(11) **EP 1 318 868 B1**
(45) Veröffentlichungstag und Bekanntmachung des Hinweises auf die Patenterteilung: **25.05.2005**
(21) Anmeldenummer: 01980398.0
(22) Anmeldetag: 17.09.2001
(51) Int. Cl.: B01J 31/18, C07F 9/655, C07F 9/6571, C07C 45/50, C07F 9/6574

(54) **VERFAHREN ZUR HYDROFORMYLIERUNG MIT KATALYSATOREN VON XANTHEN-VERBRUCKTEN LIGANDEN**
METHOD FOR HYDROFORMYLATION USING CATALYSTS OF XANTHENE-BRIDGED LIGANDS
PROCEDE D'HYDROFORMYLATION, LIGANDS A PONT DE XANTHENE ET CATALYSEUR

(30) Priorität: 18.09.2000 DE 10046026
(43) Veröffentlichungstag der Anmeldung: 18.06.2003
(73) Patentinhaber: BASF AKTIENGESELLSCHAFT, 67056 Ludwigshafen (DE)
(72) Erfinder: AHLERS, Wolfgang, 67549 Worms (DE); WIEBELHAUS, Dag, 67435 Neustadt (DE); PACIELLO, Rocco, 67098 Bad Dürkheim (DE); BARTSCH, Michael, 67433 Neustadt (DE); BAUMANN, Robert, 68161 Mannheim (DE); VOGT, Dieter, NL-5600 MB Eindhoven (NL); HEWAT, Alison, F-92400 Courbevoie (FR)
(74) Vertreter: Pohl, Michael, Dr.
(86) Internationale Anmeldenummer: PCT/EP2001/010735
(87) Internationale Veröffentlichungsnummer: WO 2002/022261

(56) Entgegenhaltungen:
- EP-A- 0 982 314
- WO-A-99/21013
- WO-A-99/46044
- WO-A-99/65606
- DE-A- 19 825 212
- US-A- 4 748 261
- DIERKES P. ET AL.: "Versatile ligands for Palladium-catalyzed asymmetric allylic alkylation" ANGEWANDTE CHEMIE. INTERNATIONAL EDITION., Bd. 37, Nr. 22, - 4. Dezember 1998 (1998-12-04) Seiten 3116-3118, XP002195282 VERLAG CHEMIE. WEINHEIM., DE ISSN: 0570-0833
- GOERTZ W. ET AL.: "Electronic effects in the nickel-catalysed hydrocyanation of styrene applying chelating phosphorus ligands with large bite angles" JOURNAL OF THE CHEMICAL SOCIETY, DALTON TRANSACTIONS., Nr. 18, - 21. September 1998 (1998-09-21) Seiten 2981-2988, XP002195283 CHEMICAL SOCIETY. LETCHWORTH., GB ISSN: 1472-7773
- GOERTZ W. ET AL.: "Asymmetric nickel- catalyzed hydrocyanation of yinylarenes by appying homochiral Xantphos ligands" CHEMISTRY - A EUROPEAN JOURNAL., Bd. 7, Nr. 8, - 17. April 2001 (2001-04-17) Seiten 1614-1618, XP002195284 VCH PUBLISHERS., US ISSN: 0947-6539
- Van der Veen L.A. et al: 'Hydroformylation of Internal Olefins to Linear Aldehydes with Novel Rhodium Catalysts', ANGEWANDTE CHEMIE. INTERNATIONAL EDITION., Bd. 38, Nr. 3, 1999, Seiten 336-338 XP000960496

## Beschreibung

Die vorliegende Erfindung betrifft ein Verfahren zur Hydroformylierung ethylenisch ungesättigter Verbindungen, wobei man als Hydroformylierungskatalysator wenigstens einen Komplex eines Metalls der VIII. Nebengruppe mit wenigstens einer Phosphor-, Arsen- oder Antimon-haltigen Verbindung als Liganden einsetzt, wobei diese Verbindung jeweils zwei ein P-, As- oder Sb-Atom und wenigstens zwei weitere Heteroatome aufweisende Gruppen gebunden an ein Xanthen-artiges Molekülgerüst aufweist.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh-, Ir-, Ru-, Pd- oder Pt-Verbindungen bzw. -komplexe eingesetzt, die zur Aktivitäts- und/oder Selektivitätsbeeinflussung mit N- oder P-haltigen Liganden modifiziert sein können. Bei der Hydroformylierungsreaktion kommt es aufgrund der möglichen CO-Anlagerung an jedes der beiden C-Atome einer Doppelbindung zur Bildung von Gemischen isomerer Aldehyde. Zusätzlich kann es auch zu einer Doppelbindungsisomerisierung kommen, d. h. zu einer Verschiebung interner Doppelbindungen auf eine terminale Position und umgekehrt.

Aufgrund der wesentlich größeren technischen Bedeutung der α-Aldehyde wird eine Optimierung der Hydroformylierungskatalysatoren zur Erzielung einer möglichst hohen Hydroformylierungsaktivität bei gleichzeitig möglichst geringer Neigung zur Bildung nicht α-ständiger Doppelbindungen angestrebt. Zudem besteht ein Bedarf an Hydroformylierungskatalysatoren, die auch ausgehend von internen linearen Olefinen in guten Ausbeuten zu α-ständigen und insbesondere n-ständigen Aldehyden führen. Hierbei muss der Katalysator sowohl die Einstellung eines Gleichgewichts zwischen internen und terminalen Doppelbindungsisomeren als auch möglichst selektiv die Hydroformylierung der terminalen Olefine ermöglichen.

Es ist bekannt, bei der Rhodium-Niederdruck-Hydroformylierung phosphorhaltige Liganden zur Stabilisierung und/oder Aktivierung des Katalysatormetalls einzusetzen. Geeignete phosphorhaltige Liganden sind z. B. Phosphine, Phosphinite, Phosphonite, Phosphite, Phosphoramidite, Phosphole und Phosphabenzole. Die derzeit am weitesten verbreiteten Liganden sind Triarylphosphine, wie z. B. Triphenylphosphin und sulfoniertes Triphenylphosphin, da diese unter den Reaktionsbedingungen eine hinreichende Stabilität besitzen. Nachteilig an diesen Liganden ist jedoch, dass im Allgemeinen nur sehr hohe Ligandenüberschüsse zufriedenstellende Ausbeuten insbesondere an linearen Aldehyden liefern.

D. Selent et al. beschreiben in Angew. Chem. Int. Ed. 39, 1639 (2000) die isomerisierende Hydroformylierung interner Olefine in Gegenwart von Rhodiumkatalysatoren, wobei als Liganden oxifunktionalisierte Bisphenylmonophosphonite eingesetzt werden. Nachteilig an diesen Katalysatoren ist ihre geringe n-Selektivität. So wurde bei der Hydroformylierung von isomeren n-Octenen n-Nonanal in einer Ausbeute von maximal 47,9 % erhalten.

Die WO-A-98/43935 beschreibt den Einsatz von Chelatliganden in Katalysatoren für die Hydroformylierung.

Haenel et al. beschreiben in Tetrahedron Letters, Band 34, Nr. 13, Seiten 2107 ff. (1993), in Tetrahedron Letters, Band 36, Nr. 1, Seiten 75 ff. (1995) und in Chem. Ber. 124, Seite 1705 ff. (1991) die Synthese von Bis-(diphenylphosphino)chelaten mit Anthracen-, Dibenzofuran-, Dibenzothiophen- und Xanthen-Grundkörpern. Ein Einsatz dieser Verbindungen in der Katalyse wird nicht beschrieben.

W. Goertz et al. beschreiben in J. Chem. Soc., Dalton Trans., 1998, S. 2981-2988 den Einsatz von Chelatphosphinen und -phosphoniten mit Thioxanthen-Grundgerüst zur Nickel-katalysierten Hydrocyanierung von Styrol. Ein Einsatz in der Hydroformylierung wird nicht beschrieben.

M. Kranenburg et al. beschreiben in Organometallics 1995, 14, Seiten 3081 bis 3089 Chelatphosphine mit Xanthen-Grundgerüst und deren Einsatz zur regioselektiven Rhodium-katalysierten Hydroformylierung. Chelatphosphonite und -phosphite sind in diesem Dokument nicht beschrieben. Nachteilig an diesen Chelatphosphinen ist, dass sie sich nicht zur isomerisierenden Hydroformylierung von internen Olefinen mit hoher α- bzw. n-Selektivität eignen.

Van der Veen et al. beschreiben in Organometallics 1999, 18, Seiten 4765 bis 4777 den Einsatz von phosphacyclischen Diphosphinen mit Xanthen-Grundgerüst als Liganden zur Rhodium-katalysierten Hydroformylierung interner Olefine. Nachteilig an diesen Katalysatoren ist ihre sehr niedrige Aktivität, die einen Einsatz in großtechnischen Verfahren unwirtschaftlich macht.

Die WO 95/30680 beschreibt zweizähnige Phosphinliganden, bei denen die Phosphoratome an ein Xanthen-Grundgerüst gebunden sein können sowie den Einsatz dieser Liganden in Katalysatoren für die Hydroformylierung. Nachteilig an diesen Katalysatoren ist, dass sie nicht zur isomerisierenden Hydroformylierung von internen Olefinen mit guter α- bzw. n-Selektivität geeignet sind.

Die EP-A-0982314 beschreibt zweizähnige carbo- oder heterocyclischen Phosphinliganden sowie ein Verfahren zur Herstellung von linearen Aldehyden durch Hydroformylierung interner Olefine unter Verwendung solcher Liganden. Nachteilig an diesen Liganden ist ihre sehr niedrige Aktivität, die einen Einsatz in großtechnischen Verfahren unwirtschaftlich macht.

W. Goertz beschreibt in seiner Dissertation zur selektiven nikkelkatalysierten Hydrocyanierung von Olefinen, Rheinisch-Westfälische Technische Hochschule Aachen, 1998, den Einsatz von Chelatphosphinen und -phosphoniten mit Xanthen-Grundgerüst als Liganden in Hydrocyanierungskatalysatoren.

W. Goertz et al. beschreiben in J. Chem. Soc., Dalton Trans., 1998, S. 2981 - 2988 elektronische Effekte bei der nickelkatalysierten Hydrocyanierung von Styrol. Die Katalysatoren basieren auf Chelatphosphinen und -phosphoniten mit Xanthen-Grundgerüst.

P. Dierkes et al. beschreiben in Angew. Chem. Int. Ed. 1998, 37, Nr. 22, S. 3116 - 3118 den Einsatz von chiralen Liganden mit Xanthenrückgrat zur palladiumkatalysierten asymmetrischen allylischen Alkylierung.

Die WO 99/46044 beschreibt Katalysatoren auf Basis von Komplexen eines Metalls der VIII. Nebengruppe, welche mindestens einen zwei- oder mehrzähnigen Phosphonitliganden umfassen, wobei die Phosphonitgruppe Teil eines 5- bis 8-gliedrigen Heterocyclus ist.

Die DE-A-198 25 212 beschreibt einen Katalysator, umfassend einen Komplex eines Metalls der VIII. Nebengruppe auf Basis eines zweizähnigen Phosphonitliganden und ein Verfahren zur Herstellung von Nitrilen.

Die US 4,748,261 beschreibt Übergangsmetall-Bisphosphit-Katalysatoren und deren Einsatz zur isomerisierenden Hydroformylierung interner Olefine.

L. A. von der Veen et al. beschreiben in Angew. Int. Ed. 1999, 38, Nr. 3, S. 336 - 338 die Hydroformylierung von internen Olefinen zu linearen Aldehyden in Gegenwart von Rhodiumkatalysatoren auf Basis von Chelatphosphinen mit Xanthen-Grundgerüst als Liganden.

Die DE-A-19827232 (WO 99/65606) beschreibt Katalysatoren auf Basis von 1-, 2- oder mehrzähnigen Phosphinitliganden, worin das Phosphor- und das Sauerstoffatom der Phosphinitgruppe Teil eines 5- bis 8-gliedrigen Heterocyclus sind sowie deren Einsatz zur Hydroformylierung und zur Hydrocyanierung. Dabei können die zweizähnigen Liganden unter anderem ein Xanthen-Grundgerüst aufweisen. Nachteilig an diesen Liganden ist, dass die in Bezug auf die α- bzw. n-Selektivität bei der isomerisierenden Hydroformylierung interner Olefine verbesserungswürdig sind.

Keines der zuvor genannten Dokumente beschreibt den Einsatz von Chelatphosphoniten und -phosphiten mit Xanthen-Grundgerüst als Liganden in Katalysatoren zur Hydroformylierung.

Der vorliegenden Erfindung liegt die Aufgabe zugrunde, ein verbessertes Verfahren zur Hydroformylierung von Verbindungen, die wenigstens eine ethylenisch ungesättigte Doppelbindung enthalten, zur verfügung zu stellen. Dabei soll bei der Hydroformylierung von α-Olefinen vorzugsweise ein möglichst hoher Anteil an α-Aldehyden, bzw. -Alkoholen erzielt werden. Insbesondere soll das Verfahren zur Hydroformylierung interner linearer Olefine bei hoher Regioselektivität zugunsten terminaler Produktaldehyde geeignet sein.

Überraschenderweise wurde nun gefunden, dass diese Aufgabe durch ein Verfahren zur Hydroformylierung gelöst wird, wobei man als Hydroformylierungskatalysator wenigstens einen Komplex eines Metalls der VIII. Nebengruppe mit mindestens einer Phosphorhaltigen Verbindung als Liganden wie in Anspruch 1 definiert einsetzt.

Gegenstand der vorliegenden Erfindung ist somit ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, wobei der Katalysator wenigstens einen Komplex eines Metalls der VIII. Nebengruppe mit mindestens einem Liganden umfasst, der ausgewählt ist unter Verbindungen der allgemeinen Formel I 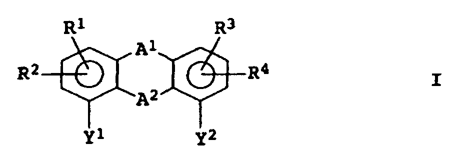 worin
- A¹ und A²: unabhängig voneinander für O, S, SiR^{a}R^{b}, NR^{c} oder CR⁵R⁶ stehen, wobei
R^{a}, R^{b}, R^{c}, R⁵ und R⁶ unabhängig voneinander für Wasserstoff, Alkyl, Cycloalkyl, Aryl oder Hetaryl stehen,
- Y¹ und Y²: unabhängig voneinander für je einen Rest der Formel OP(OR⁷)(OR⁸) stehen, worin
- R⁷ und R⁸: unabhängig voneinander für Alkyl, Cycloalkyl, Aryl oder Hetaryl stehen, welche gegebenenfalls einen, zwei oder drei Substituenten, ausgewählt unter Alkyl, Cycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, Halogen, Trifluormethyl, Nitro, Acyl oder Cyano
aufweisen, worin
R^{f}, E⁴, E⁵ und E⁶ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
x⁻ für ein Anion steht, und
y für eine ganze Zahl von 1 bis 120 steht, oder
R⁷ und R⁸ zusammen mit dem Phosphoratom und dem/den Sauerstoffatom(en), an die sie gebunden sind für einen 5- bis 8-gliedrigen Heterocyclus stehen, der gegebenenfalls zusätzlich ein-, zwei- oder dreifach mit Cycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, Halogen, Trifluormethyl, Nitro, Acyl oder Cyano, wobei R^{f}, R^{g}, E⁴, E⁵, E⁶, M⁺, X⁻ und y die zuvor angegebenen Bedeutungen besitzen, und
- R¹, R², R³ und R⁴: unabhängig voneinander für Wasserstoff, Alkyl, Cycloalkyl, Aryl, Hetaryl, COOR^{d}, COO⁻M⁺, SO₃R^{d}, SO⁻₃M⁺, NE¹E², NE¹E²E³⁺X⁻, Alkylen-NE¹E²E³⁺X⁻, OR^{d}, SR^{d}, (CHR^{e}CH₂O)ₓR^{d}, (CH₂N(E¹))ₓR^{d}, (CH₂CH₂N(E¹))ₓR^{d}, Halogen, Trifluormethyl, Nitro, Acyl oder Cyano
stehen, worin
R^{d}, E¹, E² und E³ jeweils gleiche oder verschiedene Reste, ausgewählt unter Wasserstoff, Alkyl, Cycloalkyl oder Aryl bedeuten,
R^{e} für Wasserstoff, Methyl oder Ethyl steht,
M⁺ für ein Kation steht,
X⁻ für ein Anion steht, und
x für eine ganze Zahl von 1 bis 120 steht, oder
- R¹ und/oder R³: zusammen mit zwei benachbarten Kohlenstoffatomen des Benzolkerns, an den sie gebunden sind, für ein kondensiertes Ringsystem, mit 1, 2 oder 3 weiteren Ringen stehen.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₁₂-Alkyl-, bevorzugterweise C₁-C₈-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl.

Substituierte Alkylreste weisen vorzugsweise 1, 2, 3, 4 oder 5, insbesondere 1, 2 oder 3 Substituenten, ausgewählt unter Cycloalkyl, Aryl, Hetaryl, Halogen, NE¹E², NE¹E²E³⁺, Carboxyl, Carboxylat, -SO₃H und Sulfonat auf.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy oder Halogen auf.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, Trifluormethyl, -SO₃H, Sulfonat, NE¹E², Alkylen-NE¹E², Nitro, Cyano oder Halogen auf.

Hetaryl steht vorzugsweise für Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, Carboxyl, Carboxylat, -SO₃H, Sulfonat, NE¹E², Alkylen-NE¹E², Trifluormethyl oder Halogen auf.

Die obigen Ausführungen zu Alkyl-, Cycloalkyl- und Arylresten gelten entsprechend für Alkoxy-, Cycloalkyloxy- und Aryloxyreste.

Die Reste NE¹E² und NE⁴E⁵ stehen vorzugsweise für N,N-Dimethyl, N,N-Diethyl, N,N-Dipropyl, N,N-Diisopropyl, N,N-Di-n-butyl, N,N-Di-t.-butyl, N,N-Dicyclohexyl oder N,N-Diphenyl.

Halogen steht für Fluor, Chlor, Brom und Iod, bevorzugt für Fluor, Chlor und Brom.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

Nach einer bevorzugten Ausführungsform ist in der Formel I einer der Reste Y¹ oder Y² oder sind beide Reste Y¹ und Y² ausgewählt unter solchen Resten der Formel OP(OR⁷)(OR⁸), worin R⁷ und R⁸ zusammen mit dem Phosphoratom und dem/den Sauerstoffatom(en), an die sie gebunden sind, für einen 5- bis 8-gliedrigen Heterocyclus stehen, der gegebenenfalls zusätzlich ein-, zwei- oder dreifach mit Cycloalkyl, Aryl und/oder Hetaryl anelliert ist, wobei der Heterocyclus und/oder die anellierten Gruppen unabhängig voneinander je einen, zwei, drei oder vier Substituenten, die ausgewählt unter Alkyl, Alkoxy, Halogen, Nitro, Cyano, Carboxyl, SO₃H, Sulfonat, NE⁴E⁵, Alkylen-NE⁴E⁵ und Carboxylat, tragen können.

Bevorzugt sind die Reste Y¹ und Y² ausgewählt unter Phosphitresten der allgemeinen Formel II 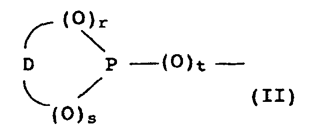 worin
- r, s und t: für 1 stehen und
- D: zusammen mit dem Phosphoratom und dem/den Sauerstoffatom(en), an die es gebunden ist, für einen 5- bis 8-gliedrigen Heterocyclus steht, der gegebenenfalls ein-, zwei- oder dreifach mit Cycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, SO₃H, Sulfonat, NE⁴E⁵, Alkylen- NE⁴E⁵, Nitro, Cyano, Carboxyl und Carboxylat, tragen können und/oder D einen, zwei oder drei Substituenten, die ausgewählt sind unter Alkyl, Alkoxy, gegebenenfalls substituiertem Cycloalkyl und gegebenenfalls substituiertem Aryl, aufweisen kann und/oder D durch 1, 2 oder 3 gegebenenfalls substituierte Heteroatome unterbrochen sein kann.

Der Rest D steht vorzugsweise für eine C₂- bis C₆-Alkylenbrücke, die 1-, oder 2-fach mit Aryl anelliert ist und/oder die einen Substituenten, der ausgewählt ist unter Alkyl, gegebenenfalls substituiertem Cycloalkyl und gegebenenfalls substituiertem Aryl, aufweisen kann und/oder die durch ein gegebenenfalls substituiertes Heteroatom unterbrochen sein kann.

Bei den anellierten Arylen der Reste D handelt es sich bevorzugt um Benzol oder Naphthalin. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE⁴E⁵, Alkylen-NE⁴E⁵, Trifluormethyl, Nitro, Carboxyl, Alkoxycarbonyl, Acyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy. Alkoxycarbonyl steht vorzugsweise für C₁- bis C₄-Alkoxycarbonyl. Halogen steht dabei insbesondere für Fluor und Chlor.

Wenn die C₂- bis C₆-Alkylenbrücke des Restes D durch 1, 2 oder 3, gegebenenfalls substituierte Heteroatome unterbrochen ist, so sind diese vorzugsweise ausgewählt unter O, S oder NR^{h}, wobei R^{h} für Alkyl, Cycloalkyl oder Aryl steht. Vorzugsweise ist die C₂- bis C₆-Alkylenbrücke des Restes D durch ein gegebenenfalls substituiertes Heteroatom unterbrochen.

Wenn die C₂- bis C₆-Alkylenbrücke des Restes D substituiert ist, so weist sie vorzugsweise 1, 2 oder 3, insbesondere 1 Substituenten auf, der/die ausgewählt ist/sind unter Alkyl, Cycloalkyl und Aryl, wobei der Arylsubstituent 1, 2 oder 3 der für Aryl genannten Substituenten tragen kann. Vorzugsweise weist die Alkylenbrücke D einen Substituenten auf, der ausgewählt ist unter Methyl, Ethyl, Isopropyl, Phenyl, p-(C₁- bis C₄-Alkyl)phenyl, bevorzugt p-Methylphenyl, p-(C₁- bis C₄-Alkoxy)phenyl, bevorzugt p-Methoxyphenyl, p-Halogenphenyl, bevorzugt p-Chlorphenyl und p-Trifluormethylphenyl.

Vorzugsweise steht der Rest D für eine C₃- bis C₆-Alkylenbrücke, die wie zuvor beschrieben anelliert und/oder substituiert und/oder durch gegebenenfalls substituierte Heteroatome unterbrochen ist. Insbesondere steht der Rest D für eine C₃- bis C₆-Alkylenbrücke, die ein- oder zweifach mit Phenyl und/oder Naphthyl anelliert ist, wobei die Phenyl- oder Naphthylgruppen 1, 2 oder 3, insbesondere 1 oder 2 der zuvor genannten Substituenten tragen können.

Vorzugsweise steht der Rest D (d. h. R⁷ und R⁸ gemeinsam) zusammen mit dem Phosphoratom und den Sauerstoffatomen, an die er gebunden ist, für einen 5- bis 8-gliedrigen Heterocyclus, wobei D (R⁷ und R⁸ gemeinsam) für einen Rest steht, der ausgewählt ist unter den Resten der Formeln II.1 bis II.5, 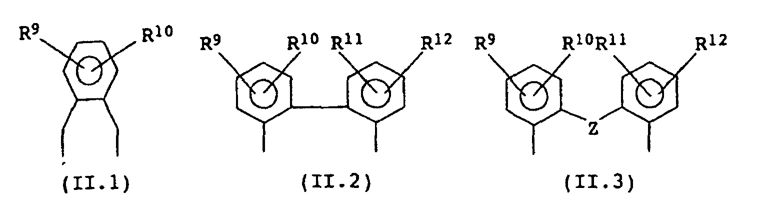 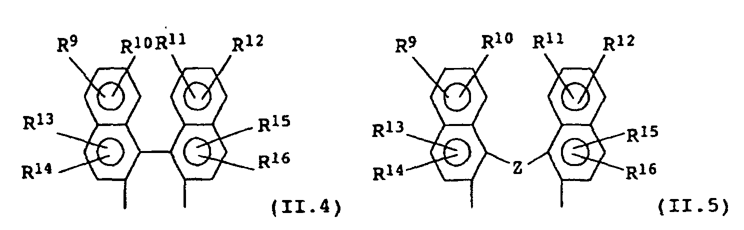 worin
- Z: für O, S oder NRⁱ steht, wobei
Rⁱ für Alkyl, Cycloalkyl oder Aryl steht,
- oder Z: für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei der für Aryl genannten Substituenten tragen kann,
- oder Z: für eine C₂- bis C₃-Alkylenbrücke steht, die durch O, S oder NRⁱ unterbrochen ist,
- R⁹, R¹⁰, R¹¹, R¹², R¹³, R¹⁴, R¹⁵ und R¹⁶: unabhängig voneinander für Wasserstoff, Alkyl, Cycloalkyl, Aryl, Alkoxy, Halogen, SO₃H, Sulfonat, NE⁴E⁵, Alkylen-NE⁴E⁵, Trifluormethyl, Nitro, Alkoxycarbonyl, Carboxyl oder Cyano stehen.

Vorzugsweise steht D für einen Rest der Formel II.1, worin R⁹ und R¹⁰ für Wasserstoff stehen.

Vorzugsweise steht D für einen Rest der Formel II.2a 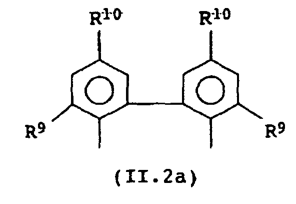 worin
- R⁹: für Wasserstoff, C₁- bis C₄-Alkyl, C₁- bis C₄-Alkoxy, SO₃H, Sulfonat, NE⁴E⁵, Alkylen-NE⁴E⁵, vorzugsweise Wasserstoff, C₁- bis C₄-Alkyl oder C₁- bis C₄-Alkoxy, insbesondere Methyl, Methoxy, Isopropyl oder tert.-Butyl, steht,
- R¹⁰: für Wasserstoff, C₁- bis C₄-Alkyl, bevorzugt Methyl, Isopropyl oder tert.-Butyl, C₁- bis C₄-Alkoxy, bevorzugt Methoxy, Fluor, Chlor oder Trifluormethyl, steht. R¹⁰ kann auch für SO₃H, Sulfonat, NE⁴E⁵ oder Alkylen-NE⁴E⁵ stehen.

Vorzugsweise steht D für einen Rest der Formel II.3a 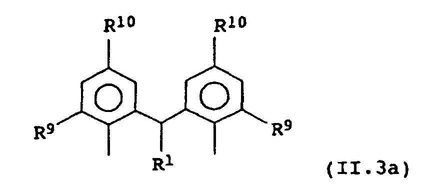 worin
- R⁹ und R¹⁰: die zuvor bei der Formel II.2a angegebenen Bedeutungen besitzen,
- R¹: für Wasserstoff, C₁- bis C₄-Alkyl, bevorzugt Methyl oder Ethyl, Phenyl, p-(C₁- bis C₄-Alkoxy)phenyl, bevorzugt p-Methoxyphenyl, p-Fluorphenyl, p-Chlorphenyl oder p-(Trifluormethyl)phenyl steht.

Vorzugsweise steht D für einen Rest der Formel II.4, worin R9, R¹⁰, R¹¹, R¹², R¹³, R¹⁴, R¹⁵ und R¹⁶ für Wasserstoff stehen.

Vorzugsweise steht D für einen Rest der Formel II.4, worin R⁹, R¹⁰, R¹¹, R¹², R¹⁴ und R¹⁶ für Wasserstoff stehen und die Reste R¹³ und R¹⁵ unabhängig voneinander für Alkoxycarbonyl, bevorzugt Methoxy-, Ethoxy-, n-Propyloxy- oder Isopropyloxycarbonyl, stehen. Insbesondere stehen die Reste R¹³ und R¹⁵ in ortho-Position zum Phosphoratom bzw. Sauerstoffatom.

Vorzugsweise steht D für einen Rest der Formel II.5, worin R⁹, R¹⁰, R¹¹, R¹², R¹³, R¹⁴, R¹⁵ und R¹⁶ für Wasserstoff stehen und Z für CR¹ steht, wobei R¹ die zuvor angegebenen Bedeutungen besitzt.

Vorzugsweise steht D für einen Rest der Formel II.5, worin R⁹, R¹⁰, R¹¹, R¹², R¹⁴ und R¹⁶ für Wasserstoff stehen, Z für CR^{h} steht und die Reste R¹³ und R¹⁵ unabhängig voneinander für Alkoxycarbonyl, bevorzugt Methoxy-, Ethoxy-, n-Propyloxy- oder Isopropyloxycarbonyl, stehen. Insbesondere stehen die Reste R¹³ und R¹⁵ in ortho-Position zum Phosphoratom bzw. Sauerstoffatom.

Nach einer weiteren bevorzugten Ausführungsform ist in der Formel I einer der Reste Y¹ oder Y² oder sind beide Reste Y¹ und Y² ausgewählt unter solchen Resten der Formel OP(OR⁷)(OR⁸), worin R⁷ und R⁸ nicht gemeinsam für einen Heterocyclus stehen.

Vorzugsweise stehen die Reste R⁷ und R⁸ unabhängig voneinander für Alkyl, Aryl oder Hetaryl, welche gegebenenfalls einen, zwei oder drei Substituenten, die ausgewählt sind unter Alkyl (nur für Aryl oder Hetaryl), Cycloalkyl, Aryl, Alkoxy, Cycloalkoxy, Aryloxy, Halogen, Trifluormethyl, Nitro, Cyano, Carboxyl, Carboxylat, Acyl, -SO₃H, Sulfonat, NE⁴E⁵ und Alkylen-NE⁴E⁵ tragen, wobei E⁴ und E⁵ gleich oder verschieden sind und ausgewählt sind unter Alkyl, Cycloalkyl und Aryl.

Vorzugsweise sind die Reste R⁷ und R⁸ unabhängig voneinander ausgewählt unter den Resten der Formeln II.6 und II.7 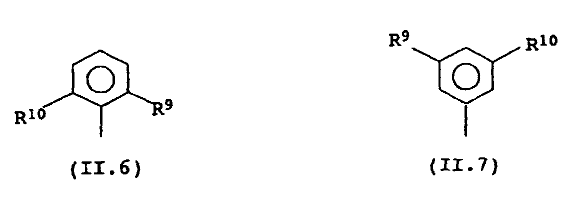 worin
- R⁹ und R¹⁰: unabhängig voneinander für Wasserstoff, C₁- bis C₄-Alkyl, COOR^{f}, COO⁻M⁺, SO₃R^{d}, SO₃⁻M⁺, NE⁴E⁵, Alkylen-NE⁴E⁵, NE⁴E⁵E⁶⁺X⁻, Alkylen-NE⁴E⁵E⁶⁺X⁻, C₁- bis C₄-Alkoxy, Halogen oder Trifluormethyl stehen, worin R^{f}, E⁴, E⁵ und E⁶ jeweils gleich oder verschieden sein können und für Wasserstoff, Alkyl, Cycloalkyl oder Aryl stehen, M⁺ für ein Kation steht und X⁻ für ein Anion steht.

Vorzugsweise stehen in den Formeln II.6 und II.7 die Reste R⁹ und R¹⁰ unabhängig voneinander für Wasserstoff, C₁- bis C₄-Alkyl oder C₁- bis C₄-Alkoxy, insbesondere Methyl, Methoxy, Isopropyl oder tert.-Butyl.

Vorzugsweise stehen A¹ und A² unabhängig voneinander für O, S und CR⁵R⁶, wobei R⁵ und R⁶ unabhängig voneinander für Wasserstoff, Alkyl, Cycloalkyl, Aryl oder Hetaryl stehen. Insbesondere stehen R⁵ und R⁶ unabhängig voneinander für Wasserstoff oder C₁- bis C₄-Alkyl, wie z. B. Methyl, Ethyl, n-Propyl, n-Butyl oder tert.-Butyl. Insbesondere stehen R⁵ und R⁶ beide für Methyl.

Vorzugsweise steht einer der Reste A¹ oder A² für 0 oder S und der andere CR⁵R⁶. Des Weiteren bevorzugt sind die Reste A¹ und A² ausgewählt unter O und S.

Die Reste R¹, R², R³ und R⁴ sind vorzugsweise ausgewählt unter Wasserstoff, Alkyl, Cycloalkyl, Aryl und Hetaryl. Bevorzugt stehen R¹ und R³ für Wasserstoff und R² und R⁴ für C₁- bis C₄-Alkyl, wie z. B. Methyl, Ethyl, n-Propyl, n-Butyl oder tert.-Butyl.

Vorzugsweise steht wenigstens einer der Reste R¹, R², R³ und/oder R⁴ für eine polare (hydrophile) Gruppe, wobei dann in der Regel wasserlösliche Katalysatoren resultieren. Bevorzugt sind die polaren Gruppen ausgewählt unter COOR^{d}, COO⁻M⁺, SO₃R^{d}, SO⁻₃M⁺, NE¹E², Alkylen-NE¹E², NE¹E²E³⁺X⁻, Alkylen-NE¹E²E³⁺X⁻, OR^{d}, SR^{d}, (CHR^{e}CH₂O)ₓR^{d} oder (CH₂CH₂N(E¹))ₓR^{d}, worin R^{d}, E¹, E², E³, R^{d}, R^{e}, M⁺, X⁻ und x die zuvor angegebenen Bedeutungen besitzen.

Besonders bevorzugt stehen R¹, R², R³ und R⁴ für Wasserstoff.

Wenn R¹ und/oder R³ für ein ankondensiertes Ringsystem stehen, so handelt es sich bevorzugt um Benzol- oder Naphthalinringe. Ankondensierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE⁴E⁵, Alkylen-NE⁴E⁵, Trifluormethyl, Nitro, Carboxyl, Alkoxycarbonyl, Acyl und Cyano. Ankondensierte Naphthaline sind vorzugsweise unsubstituiert oder weisen im nicht anellierten Ring und/oder im anellierten Ring jeweils 1, 2 oder 3, insbesondere 1 oder 2 der zuvor bei den ankondensierten Benzolringen genannten Substituenten auf.

Bevorzugt steht M⁺ für ein Alkalimetallkation, wie z. B. Li⁺, Na⁺ oder K⁺, NH₄⁺ oder eine quartäre Ammonium-Verbindung, wie sie durch Protonierung oder Quaternierung von Aminen erhältlich ist.

Bevorzugt steht X⁻ für Halogenid, besonders bevorzugt Cl⁻ und Br⁻.

Nach einer bevorzugten Ausführungsform des erfindungsgemäßen Verfahrens wird ein Hydroformylierungskatalysator eingesetzt, wobei die Verbindung der Formel I ausgewählt ist unter Verbindungen der Formeln I.1 und I.2 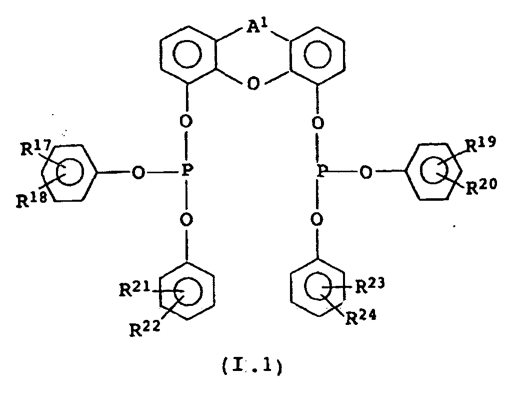 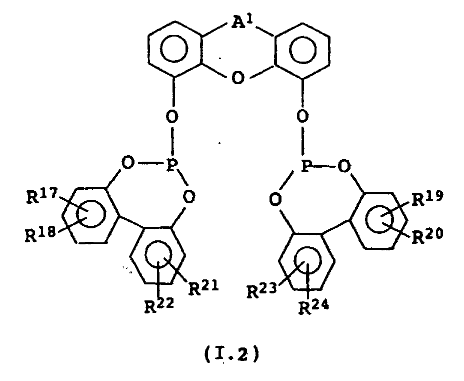 worin
- A¹: für O, S oder CR⁵R⁶ steht, wobei R⁵ und R⁶ unabhängig voneinander für Wasserstoff oder C₁- bis C₄-Alkyl, insbesondere Methyl oder tert.-Butyl, stehen,
- R¹⁷, R¹⁸, R¹⁹, R²⁰, R²¹, R²², R²³ und R²⁴: unabhängig voneinander für Wasserstoff, Alkyl, bevorzugt C₁- bis C₄-Alkyl, insbesondere Methyl oder tert.-Butyl, Cycloalkyl, Aryl, Hetaryl, COOR^{d}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, bevorzugt C₁- bis C₄-Alkoxy, insbesondere Methoxy, SR^{f}, (CHR^{g}CH₂O)_{y}R^{f}, (CH₂N(E⁴))_{y}R^{f}, (CH₂CH₂N(E⁴))_{y}R^{f}, Halogen, Trifluormethyl, Nitro, Acyl oder Cyano
stehen, worin
- R^{f}, E⁴, E⁵ und E⁶: jeweils gleiche oder verschiedene Reste, ausgewählt unter Wasserstoff, Alkyl, bevorzugt C₁- bis C₄-Alkyl, insbesondere Methyl und tert.-Butyl, Alkoxy, bevorzugt Methoxy, Cycloalkyl oder Aryl bedeuten,
- R^{g}: für Wasserstoff, Methyl oder Ethyl steht,
- M⁺: für ein Kation steht,
- X⁻: für ein Anion steht, und
- y: für eine ganze Zahl von 1 bis 120 steht.

Die erfindungsgemäß eingesetzten Katalysatoren können einen oder mehrere der Verbindungen der allgemeinen Formel I als Liganden aufweisen. Zusätzlich zu den zuvor beschriebenen Liganden der allgemeinen Formel I können Sie noch wenigstens einen weiteren Liganden, der ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit- und Phosphitliganden aufweisen.

Bevorzugt handelt es sich bei dem Metall der VIII. Nebengruppe um Cobalt, Ruthenium, Rhodium, Palladium, Platin, Osmium oder Iridium und insbesondere um Cobalt, Rhodium, Ruthenium und Iridium.

Die Herstellung der erfindungsgemäß eingesetzten Verbindungen der Formel I kann z. B. ausgehen von einer Verbindung der Formel I.a 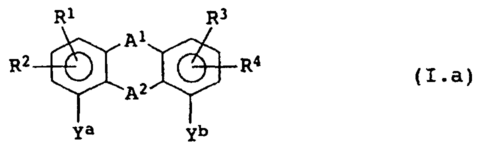 worin
- Y^{a} und Y^{b}: unabhängig voneinander für Reste Y¹ oder Y², wie zuvor definiert, stehen, oder Y^{a} und Y^{b} unabhängig voneinander für Halogen, OH, OC(O)CF₃ oder SO₃Me mit Me = Wasserstoff, Li, Na oder K, stehen, wobei Y^{a} und/oder Y^{b} auch für Wasserstoff stehen kann, wenn jeweils einer der Reste R² und/oder R⁴ Wasserstoff, eine Alkoxygruppe oder eine Alkoxycarbonylgruppe bedeutet, die sich in der ortho-Position von Y^{a} und/oder Y^{b} befindet, und
- A¹, A², R¹, R², R³ und R⁴: die zuvor angegebenen Bedeutungen besitzen.

Die Funktionalisierung der Reste Y^{a} und Y^{b} zu den Resten X¹ und Y² kann in Analogie zu bekannten Verfahren erfolgen. Beispielsweise kann man Verbindungen der Formel I.a, worin Y^{a} und Y^{b} für Halogen, vorzugsweise Chlor, stehen, zunächst lithiieren und das dabei gebildete Zwischenprodukt mit einer Verbindung, die am Phosphoratom ein Halogenatom, vorzugsweise ein Chloratom trägt, beispielsweise eine Verbindung der Formel Cl-P(OR⁷)₂, Cl-P(OR⁷)(OR⁸) oder Cl-P(OR⁷)₂, umsetzen. Die Herstellung der erfindungsgemäßen Verbindungen I, worin Y¹ und Y² für einen Rest der Formel II mit t = 0 stehen, erfolgt beispielsweise durch Umsetzung von I.a mit Verbindungen der Formel II.a gemäß folgendem Schema, 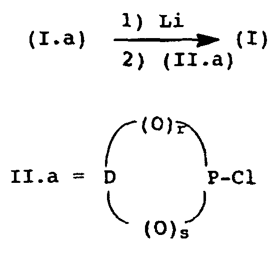 wobei r, s und D die zuvor für die Verbindungen der Formel II angegebenen Bedeutungen besitzen.

Anstelle von Verbindungen der Formel I.a mit Y^{a} = Y^{b} = Halogen könen auch solche Verbindungen I.a mit Y^{a} = Y^{b} = Wasserstoff lithiiert werden, in denen in der Orthoposition von Y^{a} und Y^{b} sich jeweils Wasserstoff, eine Alkoxygruppe oder Alkoxycarbonylgruppe befindet. Derartige Reaktionen sind unter dem Begriff "ortho-Lithiierung" in der Literatur beschrieben (siehe z.B. D. W. Slocum, J. Org. Chem., 1976, 41, 3652-3654; J. M. Mallan, R. L. Bebb, Chem. Rev., 1969, 693ff; V. Snieckus, Chem. Rev., 1980, 6, 879-933). Die dabei erhaltenen Organolithiumverbindungen können dann mit den Phosphorhalogenverbindungen in der oben angegebenen Weise zu den Zielverbindungen I umgesetzt werden.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für eine Phosphor-, Arsen- oder Antimon-haltige Verbindung der Formel I und q, x, y, z für ganze Zahlen, abhängig von der Wertigkeit und Art des Metalls sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 2 bis 5. Dabei können die Komplexe gewünschtenfalls zusätzlich noch mindestens einen der zuvor beschriebenen weiteren Liganden aufweisen.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man z. B. wenigstens eine Verbindung der allgemeinen Formel I, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltacetat, Cobaltethylhexanoat, Cobaltnaphthanoat, sowie der Cobalt-Caprolactamat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weitere geeignete Verbindungen des Cobalts, Rhodiums, Rutheniums und Iridiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z. B. BF₃, AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Folgeprodukte der Aldehyde. Weitere Lösungsmittel sind Ester aliphatischer Carbonsäuren mit Alkanolen, beispielsweise Essigester oder Texanol™, Ether wie tert.-Butylmethylether und Tetrahydrofuran. Bei ausreichend hydrophilisierten Liganden können auch Alkohole, wie Methanol, Ethanol, n-Propanol, Isopropanol, n-Butanol, Isobutanol, Ketone, wie Aceton und Methylethylketon etc., eingesetzt werden. Ferner können als Lösungsmittel auch sogenannten "Ionic Liquids" verwendet werden. Hierbei handelt es sich um flüssige Salze, beispielsweise um N,N'-Dialkylimidazoliumsalze wie die N-Butyl-N'-methylimidazoliumsalze, Tetraalkylammoniumsalze wie die Tetra-n-butylammoniumsalze, N-Alkylpyridiniumsalze wie die n-Butylpyridiniumsalze, Tetraalkylphosphoniumsalze wie die Trishexyl(tetradecyl)phosphoniumsalze, z.B. die Tetrafluoroborate, Acetate, Tetrachloroaluminate, Hexafluorophosphate, Chloride und Tosylate.

Weiterhin ist es möglich die Umsetzungen auch in Wasser oder wässrigen Lösungsmittelsystemen, die neben Wasser ein mit Wasser mischbares Lösungsmittel, beispielsweise einen Alkohol wie Methanol, Ethanol, n-Propanol, Isopropanol, n-Butanol, Isobutanol, ein Keton wie Aceton und Methylethylketon oder ein anderes Lösungsmittel enthalten. Zu diesem Zweck setzt man Liganden der Formel I ein, die mit polaren Gruppen, beispielsweise ionischen Gruppen wie SO₃Me, CO₂Me mit Me = Na, K oder NH₄ oder wie N(CH₃)₃⁺ modifiziert sind. Die Umsetzungen erfolgen dann im Sinne einer Zweiphasenkatalyse, wobei der Katalysator sich in der wässrigen Phase befindet und Einsatzstoffe und Produkte die organische Phase bilden. Auch die Umsetzung in den "Ionic Liquids" kann als Zweiphasenkatalyse ausgestaltet sein.

Das Molmengenverhältnis von phosphorhaltigem Ligand zu Metall der VIII. Nebengruppe liegt im Allgemeinen in einem Bereich von etwa 1:1 bis 1000:1.

Als Substrate für das, erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen etc.

Geeignete verzweigte, interne Olefine sind vorzugsweise C₄- bis C₂₀-Olefine, wie 2-Methyl-2-buten, 2-Methyl-2-penten, 3-Methyl-2-penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen Gemische etc.

Geeignete zu hydroformylierende Olefine sind weiterhin C₅- bis C₈-Cycloalkene, wie Cyclopenten, Cyclohexen, Cyclohepten, Cycloocten und deren Derivate, wie z. B. deren C₁- bis C₂₀-Alkylderivate mit 1 bis 5 Alkylsubstituenten. Geeignete zu hydroformylierende Olefine sind weiterhin Vinylaromaten, wie Styrol, α-Methylstyrol, 4-Isobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₁- bis C₂₀-Alkenole, -Alkendiole und -Alkadienole, wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, Vinylcyclohexen, Dicyclopentadien, 1,5,9-Cyclooctatrien sowie Butadienhomo- und -copolymere.

Bevorzugt ist die zur Hydroformylierung eingesetzte ungesättigte Verbindung ausgewählt unter internen linearen Olefinen und Olefingemischen, die wenigstens ein internes lineares Olefin enthalten. Geeignete lineare (geradkettige) interne Olefine sind vorzugsweise C₄- bis C₂₀-Olefine, wie 2-Buten, 2-Penten, 2-Hexen, 3-Hexen, 2-Hepten, 3-Hepten, 2-Octen, 3-Octen, 4-Octen etc. und Mischungen davon.

Vorzugsweise wird in dem erfindungsgemäßen Hydroformylierungsverfahren ein großtechnisch zugängiges Olefingemisch eingesetzt, das insbesondere wenigstens ein internes lineares Olefin enthält. Dazu zählen z. B. die durch gezielte Ethen-Oligomerisierung in Gegenwart von Alkylaluminiumkatalysatoren erhaltenen Ziegler-Olefine. Dabei handelt es sich im Wesentlichen um unverzweigte Olefine mit endständiger Doppelbindung und gerader Kohlenstoffatomanzahl. Dazu zählen weiterhin die durch Ethen-Oligomerisierung in Gegenwart verschiedener Katalysatorsysteme erhaltenen Olefine, z. B. die in Gegenwart von Alkylaluminiumchlorid/Titantetrachlorid-Katalysatoren erhaltenen, überwiegend linearen α-Olefine und die in Gegenwart von Nickel-Phosphinkomplex-Katalysatoren nach dem Shell Higher Olefin Process (SHOP) erhaltenen α-Olefine. Geeignete technisch zugängige Olefingemische werden weiterhin bei der Paraffin-Dehydrierung entsprechender Erdölfraktionen, z. B. der sog. Petroleum- oder Dieselölfraktionen, erhalten. Zur Überführung von Paraffinen, vorwiegend von n-Paraffinen in Olefine, werden im Wesentlichen drei Verfahren eingesetzt:
- thermisches Cracken (Steamcracken),
- katalytisches Dehydrieren und
- chemisches Dehydrieren durch Chlorieren und Dehydrochlorieren.

Dabei führt das thermische Cracken überwiegend zu α-Olefinen, während die anderen Varianten Olefingemische ergeben, die im Allgemeinen auch größere Anteile an Olefinen mit innenständiger Doppelbindung aufweisen. Geeignete Olefingemische sind weiterhin die bei Metathese- bzw. Telomerisationsreaktionen erhaltenen Olefine. Dazu zählen z. B. die Olefine aus dem Phillips-Triolefin-Prozess, einem modifizierten SHOP-Prozess aus Ethylen-Oligomerisierung, Doppelbindungs-Isomerisierung und anschließender Metathese (Ethenolyse).

Geeignete in dem erfindungsgemäßen Hydroformylierungsverfahren einsetzbare technische Olefingemische sind weiterhin ausgewählt unter Dibutenen, Tributenen, Tetrabutenen, Dipropenen, Tripropenen, Tetrapropenen, Mischungen von Butenisomeren, insbesondere Raffinat II, Dihexenen, Dimeren und Oligomeren aus dem Dimersol®-Prozess von IFP, Octolprozess® von Hüls, Polygasprozess etc.

Bevorzugt ist ein Verfahren, das dadurch gekennzeichnet ist, dass der Hydroformylierungskatalysator in situ hergestellt wird, wobei man mindestens eine Verbindung der Formel I, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von Phosphor-, Arsen- oder Antimon-haltigen Verbindungen eine Umsetzung in einem Bereich niedriger Drücke, wie etwa im Bereich von 1 bis 100 bar.

Die erfindungsgemäß eingesetzten und die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Die zuvor beschriebenen, erfindungsgemäßen Katalysatoren, die chirale Verbindungen der allgemeinen Formel I umfassen, eignen sich zur enantioselektiven Hydroformylierung.

Die zuvor beschriebenen Katalysatoren können auch in geeigneter Weise, z.B. durch Anbindung über als Ankergruppen geeignete funktionelle Gruppen, Adsorption, Pfropfung, etc. an einen geeigneten Träger, z.B. aus Glas, Kieselgel, Kunstharzen etc., immobilisiert werden. Sie eignen sich dann auch für einen Einsatz als Festphasenkatalysatoren.

Die Hydroformylierungsaktivität von Katalysatoren auf Basis von Phosphinliganden der Formel I ist überraschenderweise in der Regel höher als die Isomerisierungsaktivität bezüglich der Bildung mittenständiger Doppelbindungen. vorteilhafterweise zeigen die erfindungsgemäßen und die erfindungsgemäß eingesetzten Katalysatoren bei der Hydroformylierung von α-Olefinen eine hohe Selektivität zugunsten der α-Aldehyde bzw. -Alkohole. Zudem werden im Allgemeinen auch bei der Hydroformylierung von internen linearen Olefinen (isomerisierende Hydroformylierung) gute Ausbeuten an α-Aldehyden bzw. -Alkoholen und insbesondere auch an n-Aldehyden bzw. -Alkoholen erhalten. Weiterhin weisen diese Katalysatoren im Allgemeinen eine hohe Stabilität unter den Hydroformylierungsbedingungen auf, so dass mit Ihnen in der Regel längere Katalysatorstandzeiten erzielt werden, als mit aus dem Stand der Technik bekannten Katalysatoren auf Basis herkömmlicher Chelatliganden. Vorteilhafter Weise zeigen die erfindungsgemäßen und erfindungsgemäß eingesetzten Katalysatoren weiterhin eine hohe Aktivität, so dass in der Regel die entsprechenden Aldehyde, bzw. Alkohole in guten Ausbeuten erhalten werden. Bei der Hydroformylierung von α-Olefinen sowie von innenständigen, linearen Olefinen zeigen Sie zudem eine sehr geringe Selektivität zum Hydrierprodukt des eingesetzten Olefins.

## Patentansprüche

1. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Hydroformylierungskatalysators, wobei der Katalysator wenigstens einen Komplex eines Metalls der VIII. Nebengruppe mit mindestens einem Liganden umfasst, der ausgewählt ist unter Verbindungen der allgemeinen Formel I 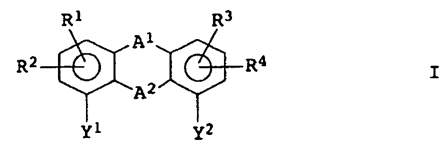 worin
A¹ und A² unabhängig voneinander für O, S, SiR^{a}R^{b}, NR^{c} oder CR⁵R⁶ stehen, wobei
R^{a}, R^{b}, R^{c}, R⁵ und R⁶ unabhängig voneinander für Wasserstoff, Alkyl, Cycloalkyl, Aryl oder Hetaryl stehen,
Y¹ und Y² unabhängig voneinander für je einen Rest der Formel OP(OR⁷)(OR⁸) stehen, worin
R⁷ und R⁸ unabhängig voneinander für Alkyl, Cycloalkyl, Aryl oder Hetaryl stehen, welche gegebenenfalls einen, zwei oder drei Substituenten, ausgewählt unter Alkyl, Cycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, Halogen, Trifluormethyl, Nitro, Acyl oder Cyano
aufweisen, worin
R^{f}, E⁴, E⁵ und E⁶ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
X⁻ für ein Anion steht, und
y für eine ganze Zahl von 1 bis 120 steht, oder
R⁷ und R⁸ zusammen mit dem Phosphoratom und dem/den Sauerstoffatom(en), an die sie gebunden sind für einen 5- bis 8-gliedrigen Heterocyclus stehen, der gegebenenfalls zusätzlich ein-, zwei- oder dreifach mit Cycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, Halogen, Trifluormethyl, Nitro, Acyl oder Cyano, wobei R^{f}, R^{g}, E⁴, E⁵, E⁶, M⁺, X⁻ und y die zuvor angegebenen Bedeutungen besitzen, und
R¹, R², R³ und R⁴ unabhängig voneinander für Wasserstoff, Alkyl, Cycloalkyl, Aryl, Hetaryl, COOR^{d}, COO⁻M⁺, SO₃R^{d}, SO⁻₃M⁺, NE¹E², NE¹E²E³⁺X⁻, Alkylen-NE¹E²E³⁺X⁻, OR^{d}, SR^{d}, (CHR^{e}CH₂O)ₓR^{d}, (CH₂N(E¹))ₓR^{d}, (CH₂CH₂N(E¹))ₓR^{d}, Halogen, Trifluormethyl, Nitro, Acyl oder Cyano
stehen, worin
R^{d}, E¹, E² und E³ jeweils gleiche oder verschiedene Reste, ausgewählt unter Wasserstoff, Alkyl, Cycloalkyl oder Aryl bedeuten,
R^{e} für Wasserstoff, Methyl oder Ethyl steht,
M⁺ für ein Kation steht,
X⁻ für ein Anion steht, und
x für eine ganze Zahl von 1 bis 120 steht, oder
R¹ und/oder R³ zusammen mit zwei benachbarten Kohlenstoffatomen des Benzolkerns, an den sie gebunden sind, für ein kondensiertes Ringsystem, mit 1, 2 oder 3 weiteren Ringen stehen.

2. Verfahren nach Anspruch 1, wobei die Verbindung der Formel I ausgewählt ist unter Verbindungen der Formel I.1 und I.2 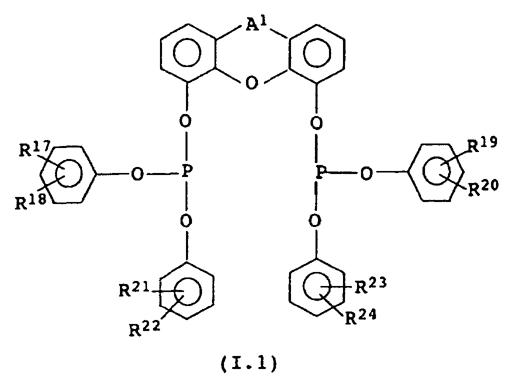 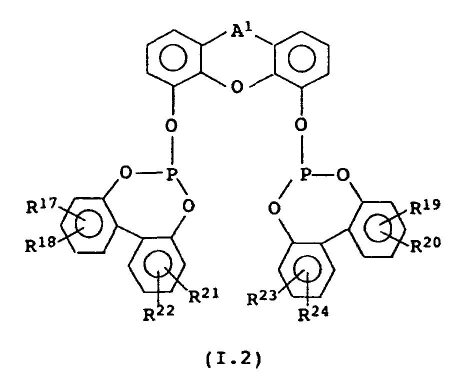 worin
A¹ für O, S oder CR⁵R⁶ steht, wobei R⁵ und R⁶ unabhängig voneinander für Wasserstoff oder C₁- bis C₄-Alkyl stehen,
R¹⁷, R¹⁸, R¹⁹, R²⁰, R²¹, R²², R²³ und R²⁴ unabhängig voneinander für Wasserstoff, Alkyl, Cycloalkyl, Aryl, Hetaryl, COOR^{f}, COO⁻M⁺, SO₃R^{f}, SO⁻₃M⁺, NE⁴E⁵, Alkylen-NE⁴E⁵, NE⁴E⁵E⁶⁺X⁻, Alkylen-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, Halogen, Trifluormethyl, Nitro, Acyl oder Cyano
stehen, worin
R^{f}, E⁴, E⁵ und E⁶ jeweils gleiche oder verschiedene Reste, ausgewählt unter Wasserstoff, Alkyl, Cycloalkyl oder Aryl bedeuten,
R^{g} für Wasserstoff, Methyl oder Ethyl steht,
M⁺ für ein Kation steht,
X⁻ für ein Anion steht, und
y für eine ganze Zahl von 1 bis 120 steht.

3. Verfahren nach einem der vorhergehenden Ansprüche, wobei das Metall der VIII. Nebengruppe ausgewählt ist unter Cobalt, Ruthenium, Iridium, Rhodium, Palladium und Platin.

4. Verfahren nach einem der vorhergehenden Ansprüche, wobei der Katalysator zusätzlich wenigstens einen weiteren Liganden, ausgewählt unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit und Phosphitliganden aufweist.

5. Verfahren nach einem der vorhergehenden Ansprüche, wobei die zur Hydroformylierung eingesetzte ungesättigte Verbindung ausgewählt ist unter internen linearen Olefinen und Olefingemischen, die wenigstens ein internes lineares Olefin enthalten.

## Claims

1. A process for the hydroformylation of compounds containing at least one ethylenically unsaturated double bond by reaction with carbon monoxide and hydrogen in the presence of a hydroformylation catalyst comprising at least one complex of a metal of transition group VIII with at least one ligand selected from among compounds of the general formula I 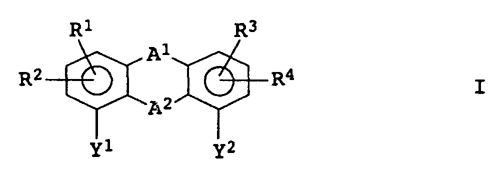 where
A¹ and A² are each, independently of one another, 0, S, SiR^{a}R^{b}, NR^{c} or CR⁵R⁶, where
R^{a}, R^{b}, R^{c}, R⁵ and R⁶ are each, independently of one another, hydrogen, alkyl, cycloalkyl, aryl or hetaryl,
Y¹ and Y² are each, independently of one another, a radical of the formula OP(OR⁷)(OR⁸), where
R⁷ and R⁸ are each, independently of one another, alkyl, cycloalkyl, aryl or hetaryl which may bear one, two or three substituents selected from among alkyl, cycloalkyl, aryl, hetaryl, COOR^{f}, COO⁻M⁺, SO₃R^{f}, SO⁻₃M⁺, NE⁴E⁵, alkylene-NE⁴E⁵, NE⁴E⁵E⁶⁺X⁻, alkylene-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, halogen, trifluoromethyl, nitro, acyl and cyano,
where
R^{f}, E⁴, E⁵ and E⁶ are identical or different radicals selected from among hydrogen, alkyl, cycloalkyl or aryl,
R^{g} is hydrogen, methyl or ethyl,
M⁺ is a cation,
X⁻ is an anion, and
y is an integer from 1 to 120, or
R⁷ and R⁸ together with the phosphorus atom and the oxygen atom(s) to which they are bound form a 5- to 8-membered heterocycle to which one, two or three cycloalkyl, aryl or hetaryl groups may additionally be fused, where the heterocycle and, if present, the fused-on groups may each bear, independently of one another, one, two, three or four substituents selected from among alkyl, cycloalkyl, aryl, hetaryl, COOR^{f}, COO⁻M⁺, SO₃R^{f}, SO⁻₃M⁺, NE⁴E⁵, alkylene-NE⁴E⁵, NE⁴E⁵E⁶⁺X⁻, alkylene-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, halogen, trifluoromethyl, nitro, acyl and cyano, where R^{f}, R^{g}, E⁴, E⁵, E⁶, M⁺, X⁻ and y are as defined above, and
R¹, R², R³ and R⁴ are each, independently of one another, hydrogen, alkyl, cycloalkyl, aryl, hetaryl, COOR^{d}, COO⁻M⁺, SO₃R^{d}, SO⁻₃M⁺, NE¹E², NE¹E²E³⁺X⁻, alkylene-NE¹E²E³⁺X⁻, OR^{d}, SR^{d}, (CHR^{e}CH₂O)ₓR^{d}, (CH₂N(E¹))ₓR^{d}, (CH₂CH₂N(E¹))ₓR^{d}, halogen, trifluoromethyl, nitro, acyl or cyano,
where
R^{d}, E¹, E² and E³ are identical or different radicals selected from among hydrogen, alkyl, cycloalkyl and aryl,
R^{e} is hydrogen, methyl or ethyl,
M⁺ is a cation,
X⁻ is an anion, and
x is an integer from 1 to 120, or
R¹ and/or R³ together with two adjacent carbon atoms of the benzene ring to which they are bound form a fused ring system having 1, 2 or 3 further rings.

2. A process as claimed in claim 1, wherein the compound of the formula I is selected from among compounds of the formulae I.1 and I.2 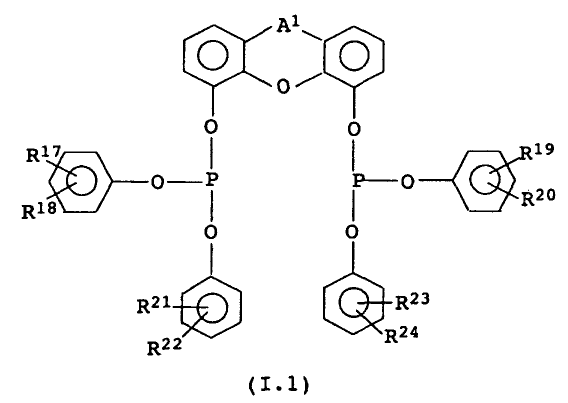 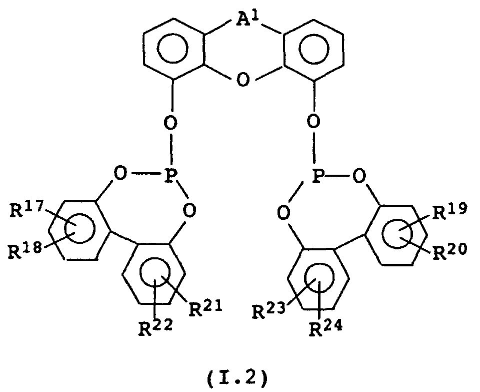 where
A¹ is O, S or CR⁵R⁶, where R⁵ and R⁶ are each, independently of one another, hydrogen or C₁-C₄-alkyl,
R¹⁷, R¹⁸, R¹⁹, R²⁰, R²¹, R²², R²³ and R²⁴ are each, independently of one another, hydrogen, alkyl, cycloalkyl, aryl, hetaryl, COOR^{f}, COO⁻M⁺, SO₃R^{f}, SO⁻₃M⁺, NE⁴E⁵, alkylene-NE⁴E⁵, NE⁴E⁵E⁶⁺X⁻, alkylene-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, halogen, trifluoromethyl, nitro, acyl and cyano,
where
R^{f}, E⁴, E⁵ and E⁶ are identical or different radicals selected from among hydrogen, alkyl, cycloalkyl and aryl,
R^{g} is hydrogen, methyl or ethyl,
M⁺ is a cation,
X⁻ is an anion, and
y is an integer from 1 to 120.

3. A process as claimed in either of the preceding claims, wherein the metal of transition group VIII is selected from among cobalt, ruthenium, iridium, rhodium, palladium and platinum.

4. A process as claimed in any of the preceding claims, wherein the catalyst further comprises at least one additional ligand selected from among halides, amines, carboxylates, acetylacetonate, arylsulfonates and alkylsulfonates, hydride, CO, olefins, dienes, cycloolefins, nitriles, N-containing heterocycles, aromatics and heteroaromatics, ethers, PF₃, phospholes, phosphabenzenes and monodentate, bidentate and polydentate phosphine, phosphinite, phosphonite, phosphoramidite and phosphite ligands.

5. A process as claimed in any of the preceding claims, wherein the unsaturated compound used for the hydroformylation is selected from among internal linear olefins and olefin mixtures in which at least one internal linear olefin is present.

## Revendications

1. Procédé pour l'hydroformylation de composés qui présentent au moins une double liaison éthyléniquement insaturée, par transformation avec du monoxyde de carbone et de l'hydrogène, en présence d'un catalyseur d'hydroformylation, le catalyseur comprenant au moins un complexe d'un métal du VIIIème groupe secondaire avec au moins un ligand, choisi parmi les composés de formule générale I 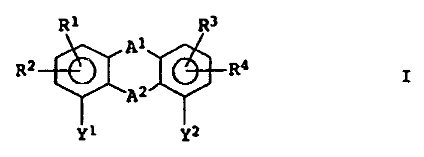 dans laquelle
A¹ et A² représentent, indépendamment l'un de l'autre, O, S, SiR^{a}R^{b}, NR^{c} ou CR⁵R⁶, où
R^{a}, R^{b}, R^{c}, R⁵ et R⁶ représentent, indépendamment l'un de l'autre, hydrogène, alkyle, cycloalkyle, aryle ou hétéroaryle,
Y¹ et Y² représentent, indépendamment l'un de l'autre, à chaque fois un radical de formule OP(OR⁷) (OR⁸) dans laquelle
R⁷ et R⁸ représentent, indépendamment l'un de l'autre, alkyle, cycloalkyle, aryle ou hétéroaryle, qui présentent le cas échéant un, deux ou trois substituants choisis parmi alkyle, cycloalkyle, aryle, hétéroaryle, COOR^{f}, COO⁻M⁺, SO₃R^{f}, SO⁻₃ M⁺, NE⁴E⁵, alkylène-NE⁴E⁵, NE⁴E⁵E⁶⁺X⁻, alkylène-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, halogène, trifluorométhyle, nitro, acyle ou cyano
dans lesquels
R^{f}, E⁴, E⁵ et E⁶ représentent à chaque fois des radicaux identiques ou différents, choisis parmi hydrogène, alkyle, cycloalkyle ou aryle,
R^{g} représente hydrogène, méthyle ou éthyle,
M⁺ représente un cation,
X⁻ représente un anion, et
y représente un nombre entier de 1 à 120, ou
R⁷ et R⁸ représentent ensemble avec l'atome de phosphore et le ou les atomes d'oxygène, auxquels ils sont liés, un hétérocycle à 5 jusqu'à 8 chaînons, qui est le cas échéant en outre annelé une, deux ou trois fois avec cycloalkyle, aryle ou hétéroaryle, l'hétérocycle et, s'ils sont présents, les groupes annelés pouvant porter, indépendamment l'un de l'autre, à chaque fois un, deux, trois ou quatre substituants, qui sont choisis parmi alkyle, cycloalkyle, aryle, hétéroaryle, COOR^{f}, COO⁻M⁺, SO₃R^{f}, SO⁻₃M⁺, NE⁴E⁵, alkylène-NE⁴E⁵, NE⁴E⁵E⁶⁺X⁻, alkylène-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, halogène, trifluorométhyle, nitro, acyle ou cyano, où R^{f}, R^{g}, E⁴, E⁵, E⁶, M⁺, X⁻ et y présentent les significations susmentionnées et
R¹, R², R³ et R⁴ représentent, indépendamment l'un de l'autre, hydrogène, alkyle, cycloalkyle, aryle, hétéroaryle, COOR^{d}, COO⁻M⁺, SO₃R^{d}, SO⁻₃M⁺, NE¹E², NE¹E²E³⁺X⁻, alkylène-NE¹E²E³⁺X⁻, OR^{d}, SR^{d}, (CHR^{e}CH₂O)ₓR^{d}, (CH₂N(E¹))ₓR^{d}, (CH₂CH₂N(E¹))ₓR^{d}, halogène, trifluorométhyle, nitro, acyle ou cyano, où
R^{d}, E¹, E² et E³ signifient à chaque fois des radicaux identiques ou différents, choisis parmi hydrogène, alkyle, cycloalkyle ou aryle,
R^{e} représente hydrogène, méthyle ou éthyle,
M⁺ représente un cation,
X⁻ représente un anion, et
x représente un nombre entier de 1 à 120, ou
R¹ et/ou R³ forment ensemble, avec deux atomes de carbone adjacents du noyau benzène, auxquels ils sont liés, un système cyclique condensé avec 1, 2 ou 3 autres cycles.

2. Procédé selon la revendication 1, le composé de formule I étant choisi parmi les composés de formule I.1 et I.2 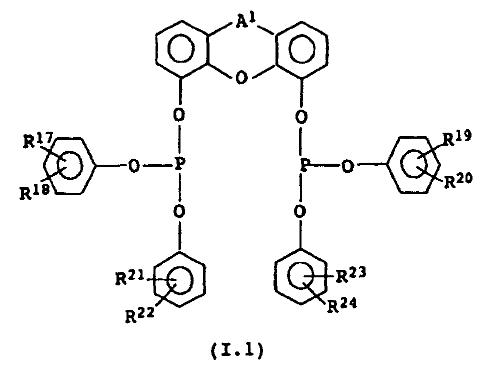 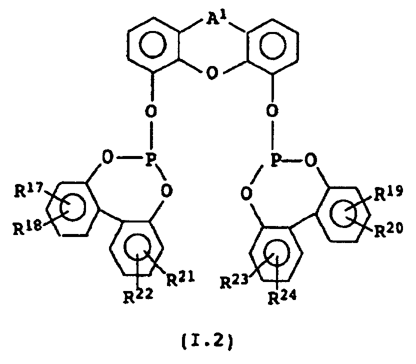 dans lesquelles
A¹ représente O, S ou CR⁵R⁶, R⁵ et R⁶ représentant, indépendamment l'un de l'autre, hydrogène ou alkyle en C₁ à C₄,
R¹⁷, R¹⁸, R¹⁹, R²⁰, R²¹, R²², R²³ et R²⁴ représentent, indépendamment l'un de l'autre, hydrogène, alkyle, cycloalkyle, aryle, hétéroaryle, COOR^{f}, COO⁻M⁺, SO₃R^{f}, SO⁻₃M⁺, NE⁴E⁵, alkylène-NE⁴E⁵, NE⁴E⁵E⁶⁺X⁻, alkylène-NE⁴E⁵E⁶⁺X⁻, OR^{f}, SR^{f}, (CHR^{g}CH₂O)_{y}R^{f}, (CH₂N(E⁴))_{y}R^{f}, (CH₂CH₂N(E⁴))_{y}R^{f}, halogène, trifluorométhyle, nitro, acyle ou cyano
dans lesquels
R^{f}, E⁴, E⁵ et E⁶ signifient à chaque fois des radicaux identiques ou différents, choisis parmi hydrogène, alkyle, cycloalkyle ou aryle,
R^{g} représente hydrogène, méthyle ou éthyle,
M⁺ représente un cation,
X⁻ représente un anion, et
y représente un nombre entier de 1 à 120.

3. Procédé selon l'une quelconque des revendications précédentes, le métal du VIIIème groupe secondaire étant choisi parmi le cobalt, le ruthénium, l'iridium, le rhodium, le palladium et le platine.

4. Procédé selon l'une quelconque des revendications précédentes, le catalyseur présentant en outre au moins un autre ligand, choisi parmi les halogénures, les amines, les carboxylates, l'acétonate d'acétyle, les sulfonates d'aryle ou d'alkyle, l'hydrure, le CO, les oléfines, les diènes, les cyclooléfines, les nitriles, les hétérocycles contenant N, les aromatiques et les hétéroaromatiques, les éthers, le PF₃, les phospholes, les phosphabenzènes ainsi que les ligands phosphine, phosphinite, phosphonite, phosphoramidite et phosphite monodentates, bidentates et polydentates.

5. Procédé selon l'une quelconque des revendications précédentes, le composé insaturé utilisé pour l'hydroformylation étant choisi parmi les oléfines linéaires internes et les mélanges d'oléfines qui contiennent au moins une oléfine linéaire interne.
